(19) Europäisches Patentamt
European Patent Office
Office européen des brevets

(11) Veröffentlichungsnummer: **0 320 764**
**A2**

# (12) EUROPÄISCHE PATENTANMELDUNG

(21) Anmeldenummer: **88120406.9**

(22) Anmeldetag: **07.12.88**

(51) Int. Cl.[4]: **C07D 231/38 , C07D 231/16 , A01N 43/56**

(30) Priorität: **17.12.87 DE 3742819**

(43) Veröffentlichungstag der Anmeldung: **21.06.89 Patentblatt 89/25**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Fürstenwerth, Hauke, Dr.**
**Unterölbach 3a**
**D-5090 Leverkusen 3(DE)**

## (54) Verfahren zur Herstellung von 5-Amino-1-Phenyl-4-nitro-pyrazolen.

(57) Die Erfindung betrifft ein neues Verfahren zur Herstellung von 5-Amino-1-phenyl-4-nitro-pyrazolen der Formel (I),

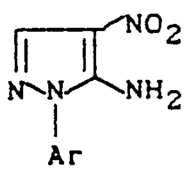

(I)

in welcher
Ar für gegebenenfalls substituiertes Phenyl steht,
dadurch gekennzeichnet, daß man Arylhydrazine der Formel (II),
$Ar - NH - NH_2$ (II)
in welcher
Ar die oben angegebene Bedeutung hat,
mit 3-Chlor-4-nitro-isothiazol der Formel (III)

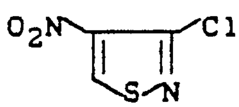

(III)

gegebenenfalls in Gegenwart einer Hilfsbase, gegebenenfalls in Gegenwart eines Verdünnungsmittels und gegebenenfalls in Gegenwart eines Reaktionshilfsmittels umsetzt.

## Verfahren zur Herstellung von 5-Amino-1-phenyl-4-nitro-pyrazolen

Die Erfindung betrifft ein neues Verfahren zur Herstellung von 5-Amino-1-phenyl-4-nitropyrazolen, welche als Herbizide verwendet werden.

Es ist bekannt, daß man 5-Amino-1-phenyl-4-nitropyrazole erhält, wenn man zunächst Arylhydrazine mit 2-Chloracrylnitril cyclisiert, in einer 2. Stufe die so erhältlichen 5-Amino-1-phenyl-pyrazole an der Aminogruppe acyliert, dann in einer 3. Stufe nitriert und schließlich in einer 4. Stufe die Acylschutzgruppe an der Aminofunktion in 5-Stellung des Pyrazolringes wieder abspaltet (vgl. z. B. EP-A 154 115 oder EP-A 224 831).

Der Nachteil des vorbekannten Verfahrens liegt in der vielstufigen Reaktionsführung, die von Aufwand- und Ausbeutebetrachtungen her als unökonomisch anzusehen ist.

Es wurde nun gefunden, daß man 5-Amino-1-phenyl-4-nitropyrazole der allgemeinen Formel (I),

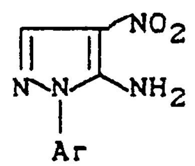

(I)

in welcher

Ar für gegebenenfalls substituiertes Phenyl steht, erhält, wenn man Arylhydrazine der Formel (II),

Ar - NH - $NH_2$ (II)

in welcher

Ar die oben angegebene Bedeutung hat,

mit 3-Chlor-4-nitro-isothiazol der Formel (III)

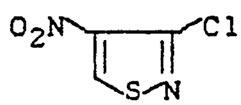

(III)

gegebenenfalls in Gegenwart einer Hilfsbase, gegebenenfalls in Gegenwart eines Verdünnungsmittels und gegebenenfalls in Gegenwart eines Reaktionshilfsmittels umsetzt.

Dabei ist es als ausgesprochen überraschend anzusehen, daß die Arylhydrazine der Formel (II) in so glatter Weise mit dem 3-Chlor-4-nitro-isothiazol der Formel (III) reagieren, da bei der Polyfunktionalität der eingesetzten Ausgangsprodukte ebenso wie der Endprodukte der Formel (I) eher mit dem Auftreten einer Vielzahl von Nebenreaktionen zu rechnen war. Zu erwarten war auch nicht, daß der als Nebenprodukt freigesetzte Schwefel aus dem Isothiazolring weder zu Nebenreaktionen noch zu Reinigungsproblemen führte. Es ist dies in der bekannten Literatur der erste und einzige Fall für die direkte Überführung einer Isothiazol-Verbindung in ein Pyrazol-Derivat.

Das erfindungsgemäße Verfahren weist gegenüber dem vorbekannten vielstufigen Herstellungsverfahren eine Reihe von Vorteilen auf. So erhält man die gewünschten Endprodukte der Formel (I) in hohen Ausbeuten in so großer Reinheit, daß in der Regel auf zusätzliche aufwendige Reinigungsoperationen verzichtet werden kann. Als weiterer Vorteil ist zu werten, daß die einstufige Reaktionsführung zu erheblichen Einsparungen von kostenintensiven Ausgangsmaterialien, Hilfsmitteln, Energie- und Abwassermengen führt, was nicht nur unter ökonomischen sondern auch unter ökologischen Aspekten eine deutliche Verbesserung gegenüber dem Stand der Technik bedeutet.

Die mit Hilfe des erfindungsgemäßen Verfahrens herstellbaren 5-Amino-1-phenyl-4-nitropyrazole sind durch die Formel (I) allgemein definiert. Bevorzugt herstellbar sind Verbindungen der Formel (I), bei welchen

Ar für gegebenenfalls einfach oder mehrfach, gleich oder verschieden substituiertes Phenyl steht, wobei als Substituenten jeweils in Frage kommen: Cyano, Nitro, Halogen, jeweils geradkettiges oder verzweigtes Alkyl, Alkoxy oder Alkoxycarbonyl mit je weils 1 bis 4 Kohlenstoffatomen, jeweils geradkettiges oder verzweigtes Halogenalkyl oder Halogenalkoxy mit jeweils 1 bis 9 gleichen oder verschiedenen Halogenatomen oder ein Rest $-S(O)_n-R^3$, wobei

$R^3$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
n für eine Zahl 0, 1 oder 2 steht.

Besonders bevorzugt herstellbar sind Verbindungen der Formel (I), bei welchen
Ar für gegebenenfalls ein- bis fünffach, gleich oder verschieden substituiertes Phenyl steht, wobei als Substituenten jeweils in Frage kommen: Cyano, Nitro, Fluor, Chlor, Brom, Iod, Methyl, Ethyl, n-oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^3$, wobei
$R^3$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht, und
n für eine Zahl 0, 1 oder 2 steht.

Ganz besonders bevorzugt herstellbar sind Verbindungen der Formel (I), bei welchen
Ar für gegebenenfalls ein- bis fünffach, gleich oder verschieden substituiertes Phenyl steht, wobei als Substituenten in Frage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl.

Verwendet man beispielsweise 2,6-Dichlor-4-trifluormethylphenylhydrazin und 3-Chlor-4-nitro-isothiazol als Ausgangsstoffe, so läßt sich der Reaktionsablauf des erfindungsgemäßen Verfahrens durch das folgende Formelschema darstellen:

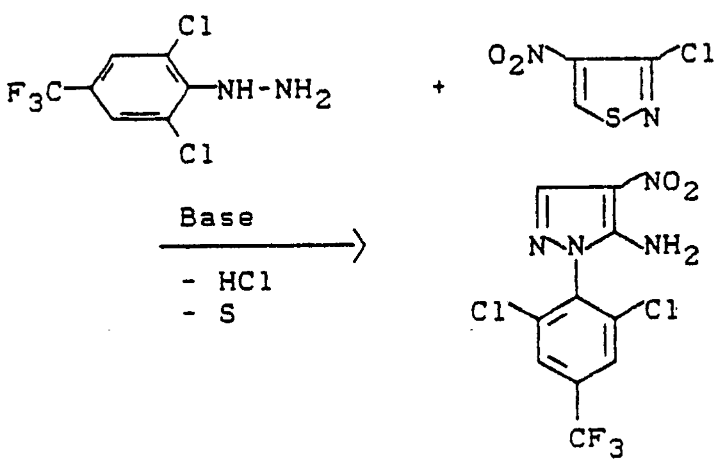


Die zur Durchführung des erfindungsgemäßen Verfahrens als Ausgangsstoffe benötigten Arylhydrazine sind durch die Formel (II) allgemein definiert. In dieser Formel (II) steht Ar vorzugsweise für diejenigen Reste, die bereits im Zusammenhang mit der Beschreibung der erfindungsgemäß herstellbaren Stoffe der Formel (I) als bevorzugt für diese Substituenten genannt wurden.

Die Arylhydrazine der Formel (II) sind bekannt oder erhältlich in Analogie zu bekannten Verfahren (vgl. z. B. EP-A 154 115 oder EP-A 224 831 oder EP-A 187 285 oder EP-A 34 945).

Das zur Durchführung des erfindungsgemäßen Verfahrens weiterhin als Ausgangsverbindung benötigte 3-Chlor-4-nitro-isothiazol der Formel (III) ist ebenfalls bekannt (vgl. US-PS 3 285 930).

Das erfindungsgemäße Verfahren wird vorzugsweise in Gegenwart einer geeigneten Hilfsbase durchgeführt.

Als solche kommen insbesondere Amine der Formel (IV),

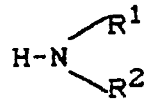 (IV)

in welcher
$R^1$ und $R^2$ unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls wietere Heteroatome enthalten kann,
in Frage.

Vorzugsweise verwendet man Amine der Formel (IV), bei welchen $R^1$ und $R^2$ unabhängig voneinander für Wasserstoff, geradkettiges oder verzweigtes Alkyl mit 1 bis 4 Kohlenstoffatomen, für Cycloalkyl mit 3 bis 7 Kohlenstoffatomen oder für jeweils gegebenenfalls ein- bis dreifach gleich oder verschieden substituiertes Phenyl oder Benzyl stehen, wobei als Substituenten in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl oder Ethoxycarbonyl, oder $R^1$ und $R^2$ gemeinsam mit dem Stickstoffatom, an welches sie gebunden sind, für einen fünf-oder sechsgliedrigen gesättigten Heterocyclus, der gege benenfalls 1 bis 3 weitere, gleich oder verschiedene Heteroatome wie insbesondere Stickstoff, Sauerstoff oder Schwefel enhalten kann, stehen. $R^1$ und $R^2$ stehen besonders bevorzugt unabhängig voneinander für Wasserstoff, Methyl, Ethyl, n- oder i-Propyl, n-, i-, s- oder t-Butyl, Cyclohexyl, Phenyl oder Benzyl oder gemeinsam mit dem Stickstoffatom, an welches sie gebunden sind, für einen 1-Pyrrolidinylrest, einen 1-Piperidinylrest oder einen 4-Morpholinylrest.

Die gegebenenfalls als Hilfsbasen zu verwendenden Amine der Formel (IV) sind allgemein bekannte Verbindungen der organischen Chemie.

Darüber hinaus können weitere anorganische oder organische Basen als Hilfsmittel zugesetzt werden. Als solche kommen alle üblichen anorganischen oder organischen Basen in Frage. Hierzu gehören beispielsweise Alkalimetallhydroxide oder Erdalkalimetallhydroxide, wie Natriumhydroxid, Kaliumhydroxid, Magnesiumhydroxid oder Calciumhydroxid, Alkalimetallcarbonate, -hydrogencarbonate oder -acetate, wie Natriumcarbonat, Kaliumcarbonat, Natriumhydrogencarbonat oder Natriumacetat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Vorzugsweise verwendet man Alkali- oder Erdalkalihydroxide, Alkalimetallcarbonate oder -hydrogencarbonate.

Als Verdünnungsmittel zur Durchführung des erfindungsgemäßen Verfahrens kommen inerte organische Lösungsmittel oder deren Gemische mit Wasser in Frage. Hierzu gehören insbesondere aliphatische, alicyclische oder aromatische, gegebenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Carbonsäuren, wie Essigsäure oder Propionsäure, Alkohole wie Methanol, Ethanol, Propanol, Butanol, Ethylenglykol, dessen Monomethyl- oder Monoethylether oder deren Gemische mit Wasser. Vorzugsweise verwendet man Alkohole oder deren Gemische mit Wasser.

Verwendet man als Hilfsbase Amine in flüssiger Form, so ist es auch möglich, diese in entsprechendem Überschuß gleichzeitig als Verdünnungsmittel einzusetzen.

Das erfindungsgemäße Verfahren kann gegebenenfalls auch in Gegenwart eines sauren Reaktionshilfsmittels durchgeführt werden. Als solche können übliche Protonen- oder Lewis-Säuren verwendet werden. Mit Vorzug verwendet man anorganische Mineralsäuren wie Salzsäure oder Schwefelsäure, aliphatische oder aromatische Carbon- oder Sulfonsäuren, wie Essigsäure, Methansulfonsäure oder p-Toluolsulfonsäure, Lewis-Säuren, wie Bortrifluorid, Eisentrichlorid, Aluminiumtrichlorid oder Zinkdichlorid oder saure Ionenaustauscher. Besonders bevorzugt verwendet man Salzsäure oder Schwefelsäure.

Die Reaktionstemperaturen können bei der Durchführung des erfindungsgemäßen Verfahrens in einem größeren Bereich variiert werden. Im allgemeinen arbeitet man bei Temperaturen zwischen -30 °C und +150 °C, vorzugsweise bei Temperaturen zwischen 20 °C und 100 °C.

Zur Durchführung des erfindungsgemäßen Verfahrens setzt man pro Mol an Arylhydrazin der Formel (II) im allgemeinen 1.0 bis 1.5 Mol, vorzugsweise 1.0 bis 1.3 Mol an 3-Chlor-4-nitro-isothiazol der Formel (III) und gegebenenfalls 1.0 bis 10.0 Mol, vorzugsweise 1.0 bis 5.0 Mol an Hilfsbase sowie gegebenenfalls 1.0 bis 20.0 Mol, vorzugsweise 1.0 bis 10.0 Mol an saurem Reaktionshilfsmittel ein.

Verwendet man als Hilfsbase Amine der Formel (IV), so setzt man diese zunächst mit dem 3-Chlor-4-nitro-isothiazol der Formel (III) um und gibt dann zu dem so erhältlichen Reaktionsgemisch das Arylhydrazin der Formel (II) und das saure Reaktionshilfsmittel.

Die Reaktionsdurchführung, Aufarbeitung und Isolierung der Reaktionsprodukte erfolgt nach allgemein üblichen Methoden, beispielsweise durch Entfernen des organischen Verdünnungsmittels, Ausfällen des

Reaktionsproduktes in Wasser, Absaugen und Trocknen des so erhältlichen Produktes.

Die mit Hilfe des erfindungsgemäßen Verfahrens herstellbaren 5-Amino-1-phenyl-4-nitropyrazole sind bekannte Herbizide (vgl. z. B. EP-A 154 155 oder EP-A 224 831).

## Herstellungsbeispiele

### Beispiel 1

a)

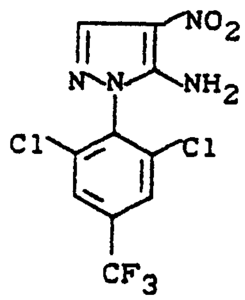

Zu 11,5 g (0.07 Mol) 3-Chlor-4-nitro-isothiazol in 50 ml Ethanol gibt man bei 0 °C bis 5 °C 12,3 g (0.14 Mol) ca. 50 %ige wäßrige Dimethylaminlösung, rührt 1 Stunde bei Raumtemperatur, gibt dann 12,25 g (0.05 Mol) 2.6-Dichlor-4-trifluormethylphenylhydrazin und 10 ml 30 %ige Salzsäure zu und erhitzt für 3 Stunden auf Rückflußtemperatur. Die abgekühlte Reaktionsmischung wird filtriert, das Filtrat im Vakuum eingeengt, der Rückstand mit 100 ml Wasser verrührt, der so entstandene Niederschlag abgesaugt, mit Wasser gewaschen und getrocknet.

Man erhält 15,6 g (91 % der Theorie) an 5-Amino-1-(2,6-dichlor-4-trifluormethylphenyl)-4-nitropyrazol vom Schmelzpunkt 190 °C.

### Beispiel 1

b)

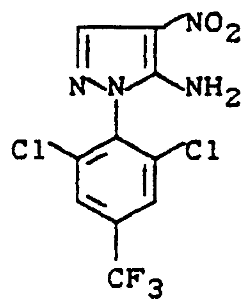

Zu 16,5 g (0.1 Mol) 3-Chlor-4-nitro-isothiazol in 200 ml Ethanol gibt man bei 0 °C bis 5 °C 22,6 g (0.22 Mol) ca. 30 %ige wäßrige Methylaminlösung, rührt 1 Stunde bei Raumtemperatur, gibt dann 17,2 g (0.07 Mol) 2.6-Dichlor-4-trifluormethylphenylhydrazin und 10 ml 30 %ige Salzsäure zu und erhitzt für 3 Stunden auf Rückflußtemperatur. Die abgekühlte Reaktionsmischung wird filtriert, das Filtrat im Vakuum eingeengt, der Rückstand mit 100 ml Wasser verrührt, der so entstandene Niederschlag abgesaugt, mit Wasser gewaschen und getrocknet.

Man erhält 21,5 g (91 % der Theorie) an 5-Amino-1-(2,6-dichlor-4-trifluormethylphenyl)-4-nitropyrazol vom Schmelzpunkt 190 °C.

In entsprechender Weise erhält man

**Beispiel 2:**

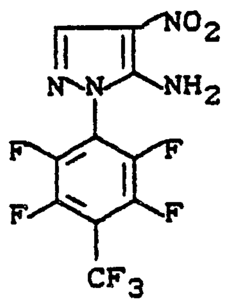

Fp. 120 °C - 122 °C
Ausbeute: 81% der Theorie

**Beispiel 3:**

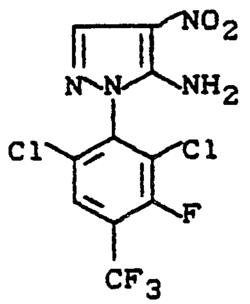

Fp. 147 - 157 °C
Ausbeute: 90% der Theorie

## Ansprüche

1. Verfahren zur Herstellung von 5-Amino-1-phenyl-4-nitropyrazolen der Formel (I),

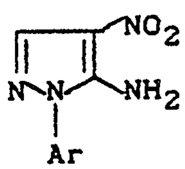

(I)

in welcher
Ar für gegebenenfalls substituiertes Phenyl steht,
dadurch gekennzeichnet, daß man Arylhydrazine der Formel (II),
$Ar - NH - NH_2$ (II)
in welcher
Ar die oben angegebene Bedeutung hat,
mit 3-Chlor-4-nitro-isothiazol der Formel (III)

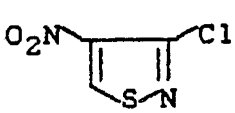

(III)

gegebenenfalls in Gegenwart einer Hilfsbase, gegebenenfalls in Gegenwart eines Verdünnungsmittels und gegebenenfalls in Gegenwart eines Reaktionshilfsmittels umsetzt.

2. Verfahren gemäß Anspruch 1, dadurch gekennzeichnet, daß man die Umsetzung bei Temperaturen zwischen -30° C und +150° C durchführt.

3. Verfahren nach Anspruch 1, dadurch gekennzeichnet, daß man pro Mol an Arylhydrazin der Formel (II) 1 bis 1,5 Mol an 3-Chlor-4-nitro-isothiazol und gegebenenfalls 1 bis 10 Mol an Hilfsbase sowie gegebenenfalls 1 bis 10 Mol an saurem Reaktionshilfsmittel einsetzt.

4. Verfahren nach Anspruch 1, dadurch gekennzeichnet, daß man pro Mol an Arylhydrazin der Formel (II) 1 bis 1,3 Mol an 3-Chlor-4-nitro-isothiazol und gegebenenfalls 1 bis 5 Mol an Hilfsbase sowie gegebenenfalls 1 bis 10 Mol an saurem Reaktionshilfsmittel einsetzt.

5. Verfahren gemäß Anspruch 1, dadurch gekennzeichnet, daß man Amine der Formel (IV),

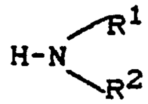

(IV)

in welcher
$R^1$ und $R^2$ unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls wietere Heteroatome enthalten kann,
als Hilfsbasen verwendet.

6. Verfahren gemäß Anspruch 5, dadurch gekennzeichnet, daß in der Formel (IV)
$R^1$ und $R^2$ unabhängig voneinander für Wasserstoff, geradkettiges oder verzweigtes Alkyl mit 1 bis 4 Kohlenstoffatomen, für Cycloalkyl mit 3 bis 7 Kohlenstoffatomen oder für jeweils gegebenenfalls ein- bis dreifach gleich oder verschieden substituiertes Phenyl oder Benzyl stehen, wobei als Substituenten in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl oder Ethoxycarbonyl, oder $R^1$ und $R^2$ gemeinsam mit dem Stickstoffatom, an welches sie gebunden sind, für einen fünf- oder sechsgliedrigen gesättigten Heterocyclus, der gegebenenfalls 1 bis 3 weitere, gleich oder verschiedene Heteroatome enthalten kann, stehen.

7. Verfahren gemäß Anspruch 1, dadurch gekennzeichnet, daß die Umsetzung in Gegenwart eines sauren Reaktionshilfsmittels durchgeführt wird.

8. Verfahren gemäß Anspruch 7, dadurch gekennzeichnet, daß man anorganische Mineralsäuren, aliphatische oder aromatische Carbonsäuren, aliphatische oder aromatische Sulfonsäuren, Lewis-Säuren oder saure Ionenaustauscher als saure Reaktionshilfsmittel einsetzt.

9. Verfahren gemäß Anspruch 1, dadurch gekennzeichnet, daß die Umsetzung in Gegenwart von Alkalimetallhydroxiden, Erdalkalimetallhydroxiden, Alkalimetallcarbonaten oder -hydrogencarbonaten oder -acetaten oder tertiären Aminen als Basen durchgeführt wird.